# DEMANDE DE BREVET EUROPEEN

(11) **EP 1 792 601 A1**
(43) Date de publication de la demande: **06.06.2007**
(21) Numéro de dépôt: 06123254.2
(22) Date de dépôt: 31.10.2006
(51) Int. Cl.: A61K 8/26, A61K 8/25, A61Q 15/00

(54) **Utilisation comme agent anti-transpirant d'une dispersion de particules colloïdales cationiques de silice; compositions anti-transpirantes; procédé cosmétique de traitement de la transpiration**

(30) Priorité: 05.12.2005 FR 0553718
(71) Demandeur: L'ORÉAL, 75008 Paris (FR)
(72) Inventeur: Lemoine, Cyril, 78210, Saint Cyr L'Ecole (FR); Ilekti, Philippe, 94700 Maison-Alfort (FR)
(74) Mandataire: Miszputen, Laurent

(57) **Abrégé**

La présente invention a pour objet l'utilisation d'une dispersion de particules colloïdales cationiques de silice modifiée par un cation multivalent dans une composition cosmétique comme actif anti-transpirant.

La présente invention concerne également des compositions anti-transpirantes comprenant au moins une dispersion de particules colloïdales cationiques de silice modifiée par un cation multivalent.

La présente invention a pour objet également un procédé cosmétique de traitement de la transpiration, consistant à appliquer sur la surface de la peau une quantité efficace d'une dispersion de particules colloïdales cationiques de silice modifiée par un cation multivalent

La présente invention a pour objet également un procédé cosmétique de traitement des odeurs corporelles, consistant à appliquer sur la surface de la peau une quantité efficace d'une dispersion de particules colloïdales cationiques de silice modifiée par un cation multivalent.

## Description

La présente invention porte sur l'utilisation d'une dispersion de particules colloïdales cationiques de silice modifiée par un cation multivalent comme actif anti-transpirant dans une composition cosmétique destiné diminuer la transpiration.

Dans le domaine cosmétique, il est bien connu d'utiliser en application topique, des produits anti-transpirants contenant des substances qui ont pour effet de limiter voire de supprimer le flux sudoral. Ces produits sont en général disponibles sous forme de roll-on, de sticks, d'aérosol ou de spray.

Les substances anti-transpirantes sont généralement constituées de sels d'aluminium ou de sels d'aluminium/zirconium. L'utilisation de ces actifs à des concentrations élevées en vue d'obtenir une bonne efficacité entraîne des difficultés de mise en formulation. De plus, ces substances présentent un potentiel irritant pour la peau.

Il existe donc le besoin de rechercher de nouveaux actifs anti-transpirants pouvant remplacer les sels d'aluminium et les sels d'aluminium/zirconium, qui soient efficaces et facilement formulables.

La Demanderesse a découvert d'une manière surprenante qu'une dispersion de particules colloïdales cationiques de silice modifiée par un cation multivalent dans un milieu physiologiquement acceptable constituait un bon agent anti-transpirant et pouvait être facilement formulée dans de nombreux produits destinés à diminuer la transpiration sans qu'il soit nécessaire d'utiliser des sels astringents classiques.

La présente invention a pour objet l'utilisation d'une dispersion de particules colloïdales cationiques de silice modifiée par un cation multivalent dans une composition cosmétique.

La présente invention concerne également des compositions anti-transpirantes comprenant au moins une dispersion de particules colloïdales de silice modifiée par un cation multivalent.

La présente invention a pour objet également un procédé cosmétique de traitement de la transpiration, consistant à appliquer sur la surface de la peau une quantité efficace d'une dispersion de particules colloïdales cationiques de silice modifiée par un cation multivalent.

Par "agent anti-transpirant", on entend toute substance qui, à elle seule, a pour effet de diminuer ou limiter le flux sudoral.

Par "composition anti-transpirante", on entend toute composition qui appliquée sur la surface de la peau, a pour effet de diminuer ou limiter le flux sudoral.

Par « cation divalent », on entend par cation ayant une valence supérieure ou égale à 2 et de préférence égale à 3.

Par "dispersion de particules colloïdales de silice " au sens de la présente invention, on entend une dispersion de particules de silice (SiO₂) ayant un diamètre moyen en nombre compris entre 3 et 150 nm, de préférence entre 5 et 30 nm, mieux, entre 10 et 25 nm. Ces particules conservent les diamètres précités dans la composition les contenant, sans s'agréger fortement, et n'ont pas de propriétés épaississantes en ce sens qu'à une concentration supérieure ou égale à 15% en poids dans l'eau, les particules colloïdales selon l'invention présentent une viscosité inférieure à 0,05 Pa.s pour un taux de cisaillement égal à 10-1s, la viscosité étant mesurée à 25°C à l'aide d'un rhéomètre RheoStress RS150 de HAAKE en configuration cône-plan, le cône ayant un diamètre de 60 mm et un angle de 2°. De manière préférée, le milieu de dispersion des particules colloïdales inorganiques est un milieu aqueux, hydroalcoolique ou alcoolique, plus préférentiellement un milieu aqueux.

Par "particules cationiques de silice modifiée par un cation multivalent" au sens de la présente invention, on entend des particules constituées d'oxyde de silicium et dont la surface a été modifiée chimiquement par des cations formant au moins une couche monomoléculaire dudit cation ; chaque cation étant reliés à la silice de telle sorte à la rendre cationique. De telles particules composites renferment généralement de 5 à 60% en poids, de préférence de 10 à 50% en poids de silice (SiO₂) et leur portion de surface recouverte de cation multivalent est généralement comprise entre 1 et 100%, de préférence entre 2 et 60%, mieux, entre 5 et 60%.

Le cation présent dans les particules colloïdales de silice modifiée peut être choisi parmi les métaux alcalino-terreux comme le calcium (Ca ²⁺), le Magnésium (Mg ²⁺), le Strontium (Sr ²⁺) le Baryum (Ba ²⁺) ; les métaux de transition comme le Titane (Ti ²⁺, Ti ³⁺, Ti ⁴⁺) , le Manganèse (Mg ²⁺, Mg ³⁺ , Mg ⁴⁺, Mg⁷⁺), Fer (Fe2+ , Fe3+), le Zirconium (Zr ⁴⁺), l'Hafnium (Hf ⁴⁺), l'Aluminum (Al ³⁺).

De préférence, le cation sera choisi parmi l'Aluminium (Al ³⁺) et/ou le Zirconium (Zr ⁴⁺) et/ou l'Hafnium (Hf ⁴⁺) et plus préférentiellement l'Aluminium (Al ³⁺).

Les particules colloïdales de silice modifiée cationiquement peuvent également comprendre en plus un contre-ion anionique stabilisant de préférence le chlore (Cl⁻).

Parmi les dispersions de particules colloïdales cationiques de silice modifiée par de l'aluminium AI 3+ conformes à l'invention, on peut citer celles disponibles dans le commerce auprès de la société GRACE sous la référence commerciale Ludox ® comme en particulier le produit LUDOX CL® (25% de silice (Si02) taille moyenne de particule environ 12 nm et stabilisé par ion CI-) de structure :

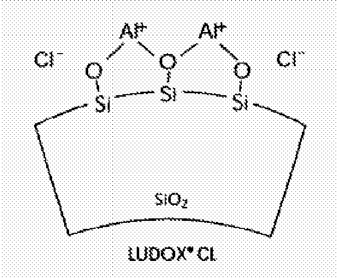

Les particules colloïdales cationiques de silice conformes à l'invention utilisées comme actifs anti-transpirants sont de préférence présents dans les compositions selon l'invention dans des quantités comprises entre 1 et 80% en poids en matière active et plus préférentiellement entre 5% et 50% et plus préférentiellement entre 10% et 50% en poids par rapport au poids total de la composition.

La composition selon l'invention a de préférence un pH inférieur à 7, mieux allant de 3 à 6,5.

La composition selon l'invention peut se présenter sous toutes les formes galéniques classiquement utilisées pour une application topique et notamment sous forme de gels aqueux, de solutions aqueuses ou hydroalcooliques. Elles peut aussi, par ajout d'une phase grasse ou huileuse, se présenter sous forme de dispersions du type lotion, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un objet de l'invention, Les compositions anti-transpirantes sont conditionnés dans un dispositif aérosol ou par un flacon pompe ; dans un dispositif muni d'une paroi ajourée telle qu'une grille ; sous forme de bâtonnets (sticks) et sont caractérisées par le fait qu'elles contiennent au moins une dispersion de particules colloïdales de silice modifiée par un cation telle que définie précédemment. Elles contiennent à cet égard les ingrédients généralement utilisés dans ce type de produits et bien connus de l'homme de l'art.

Les compositions selon l'invention destinées à l'usage cosmétique peuvent comporter au moins une phase aqueuse. Elle sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112).

La phase aqueuse desdites compositions contient de l'eau et en général d'autres solvants solubles ou miscibles dans l'eau. Les solvants solubles ou miscibles dans l'eau comprennent les mono alcools à chaîne courte par exemple en C₁-C₄ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

Les compositions selon l'invention peuvent contenir au moins une phase liquide organique non-miscible dans l'eau. Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase organique liquide organique non-miscible dans l'eau conforme à l'invention est généralement constituée d'une huile ou de mélange d'huiles et comprend au moins 80% de composés ayant une vapeur de pression ne dépassant pas la valeur 4 kPa (30 mm Hg) à 25 °C.

La phase liquide organique non-miscible dans l'eau contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones D₄, D₅ ou D₆.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les Diméthicone Copolyols.

Parmi les huiles émollientes non-volatiles utilisables dans la présente invention, on peut citer par exemple : les dérivés hydrocarbonés, les huiles minérales, les alcools gras, les esters d'alcools en C₃-C₁₈ avec des acides en C₃-C₁₈, les esters de l'acide benzoïque avec des alcools en C₁₂-C₁₈ et leurs mélanges, des polyols en C₂-C₆ choisis de préférence parmi le glycérol, le propylèneglycol ou le sorbitol, les polymères de polalkylène glycol.

Les huiles émollientes sont présentes de préférence dans des quantités allant de 1 à 50% en poids et plus préférentiellement de 5 à 40% en poids par rapport au poids total de la composition.

Selon un autre objet particulier de l'invention, la composition anti-transpirante contient au moins une dispersion de particules colloïdales cationiques de silice modifiée par un cation telle que définie ci-dessus et au moins un actif anti-transpirant.

Les actifs anti-transpirants optionnel sont choisis de préférence parmi les sels d'aluminium et/ou de zirconium ; les complexes d'hydroxychlorure de zirconium et d'hydroxychlorure d'aluminium avec un acide aminé tels que ceux décrits dans le brevet US-3792068 communément connus sous l'appellation "ZAG complexes. De tels complexes sont généralement connus sous l'appellation ZAG (lorsque l'acide aminé est la Glycine). Les complexes ZAG présentent d'ordinaire un quotient Al/Zr allant d'environ 1,67 à 12,5 et un quotient Métal/Cl allant d'environ 0,73 à 1,93. Parmi ces produits on peut citer l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.

Parmi les sels d'aluminium, on peut citer le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement l'hydroxychlorure d'aluminium commercialisé par la société REHEIS sous la dénomination REACH 301 ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF.

Les actifs anti-transpirants additionnels peuvent être présents dans la composition selon l'invention à raison d'environ 0,001 à 30% en poids par rapport à la composition totale, et de préférence à raison d'environ 0,1 à 25% en poids.

Les compositions cosmétiques selon l'invention peuvent contenir éventuellement un ou plusieurs actifs déodorants additionnels comme par exemple
- des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide ou leurs mélanges
- des sels de zinc comme le salicylate de zinc , le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoléate de zinc, glycinate de zinc, carbonate de zinc, citrate de zinc, chlorure de zinc, le laurate de zinc, l'oléate de zinc, l'orthophosphate de zinc, le stéarate de zinc, le tartrate de zinc, le lactate de zinc, l'acétate de zinc ou leurs mélanges ;

Afin d'améliorer l'efficacité anti-transpirante de la composition, on peut utiliser en plus un ou plusieurs polymères anioniques hydrosolubles comprenant un acide Bronsted en particulier ceux dérivant de l'acide maléique et/ou de l'anhydride maléique qui sont décrits dans la demande de brevet WO02/49590.

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyl-lysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges. La ou les poudres organiques peuvent être présentes par exemple en une quantité

Les compositions cosmétiques selon l'invention peuvent comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les épaississants peuvent également être cationiques comme par exemple le POLYQUATERNIUM-37 commercialisé sous la dénomination Salcare SC95 (Polyquaternium-37 (And) Mineral Oil (And) PPG-1 Trideceth-6) ou Salcare SC96 (Polyquaternium-37 (And) Propylene Glycol Dicaprylate/Dicaprate (And) PPG-1-Trideceth-6) ou d'autre polymère cationiques réticulés comme par exemple ceux de nom CTFA Copolymère Ethylacrylate / Dimethylamino Ethyl Methacrylate Cationique En Emulsion.

Les quantités de ces différents constituants pouvant être présents dans la composition cosmétique selon l'invention sont celles classiquement utilisées dans compositions anti-transpirantes.

Lorsqu'elles contiennent une phase liquide organique non-miscible dans l'eau, les compositions selon l'invention lpeuvent également contenir également un ou plusieurs agents structurants ou gélifiants de ladite phase liquide organique non-miscible dans l'eau tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'-dibutylsuccinamide) ;les organopolysiloxane élastomères tels que ceux décrits dans la demande WO97/44010.

Les compositions selon l'invention peuvent encore être pressurisées et être conditionnées dans un dispositif aérosol constitué par :
(A) un récipient comprenant une composition anti-transpirante telle que définie précédemment,
(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

Les compositions contenant le ou les polymères actifs anti-transpirants tels que définis précedemment et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

Les exemples qui suivent servent à illustrer la présente invention.

### Exemple 1

On évalue l'efficacité anti-transpirante d'une dispersion aqueuse à 20% en matière active de particules colloïdales cationiques de silice modifié par de l'Aluminium (AI ³⁺) de taille moyenne d'environ 12 nm stabilisé par du chlore à pH d'environ 4 du type LUDOX CL vendue par GRACE.

On compare cette efficacité anti-transpirante
- à celle d'une dispersion aqueuse à 20% en matière active de silice colloïdale anionique (de taille de particules 18 nm COSMO S 40) commercialisée par la société Catalysts et Chemicals
- à celle d'un chlorhydrate d'Aluminium classique CHLORHYDROL 50% USP commercialisé par la société Reheis et formulé dans un roll-on hydro-alcoolique à 10% en poids de matière active.

### PROTOCOLE

On effectue les tests sur 22 sujets

On délimite de 2 fois 8 zones (4X 5 cm²) de part et d'autre de la colonne vertébrale. A chaque zone produit correspond une zone témoin non traitée symétrique.

On applique pendant 4 jours, la dispersion colloïdale de silice alumine à raison de 75 mg ou 75 µl sur chaque zone produit correspondante. On effectue une occlusion pendant 1 heure. Les sujets restent pendant toute la période d'occlusion dans une pièce maintenue à 30°C et 50% d'humidité relative.

24H après la dernière application, on effectue un lavage du dos à l'eau pour éliminer toute trace de produit restant ; on fixe des carrés de cellulose sur les différentes zones et sudation en sauna pendant 15 minutes à 80°C.

On évalue de la quantité de sueur par pesée des carrés de cellulose avant et après sudation. On détermine le pourcentage de réduction de la sueur par rapport à la zone témoin sans produit. Un pourcentage de réduction négatif signifie que la quantité de sueur prélevée est plus élevée que la zone témoin sans produit.

On obtient avec la dispersion de particules colloïdales conformes à l'invention un pourcentage de réduction positive de la transpiration significatif de 16% par rapport à la zone témoin sans produit. Ceci montre que ladite dispersion présente une activité significative anti-transpirante. Un roll-on anti-transpirant classique du type hydro-alcoolique comprenant 15% en poids en matière active de chlorhydrate d'aluminium donne un pourcentage de réduction positif de la transpiration de 11 %.

Par contre, on obtient avec la dispersion aqueuse à pH 4 à 20% en matière active de particules colloïdales COSMO S40 une absence d'efficacité anti-transpirante, voire une augmentation de la transpiration de 13%.

## Revendications

1. Utilisation d'une dispersion de particules colloïdales cationiques de silice modifiée par un cation multivalent dans une composition cosmétique comme actif anti-transpirant.

2. Utilisation selon la revendication 1, où lesdites particules de silice ont un diamètre moyen en nombre compris entre 3 et 150 nm, de préférence entre 5 et 30 nm, mieux, entre 10 et 25 nm.

3. Utilisation selon la revendication 1 ou 2, où lesdites particules de silice renferment généralement de 5 à 60% en poids, de préférence de 10 à 50% en poids de silice (SiO₂).

4. Utilisation selon l'une quelconque des revendications 1 à 4, où lesdites particules de silice où le milieu de dispersion est un milieu aqueux, hydro-alcoolique ou alcoolique.

5. Utilisation selon la revendication 4, où le milieu de dispersion est un milieu aqueux.

6. Utilisation selon l'une quelconque des revendications 1 à 5, où lesdites particules de silice ont une portion de surface recouverte de cation comprise entre 1 et 100%, de préférence entre 10 et 60%, mieux, entre 20 et 40%.

7. Utilisation selon l'une quelconque des revendications 1 à 6, où le cation présent dans les particules colloïdales de silice modifiée est choisi parmi les métaux alcalino-terreux ; les métaux de transition et l'Aluminum (Al ³⁺).

8. Utilisation selon la revendication 7, où le cation est l'Aluminium (Al ³⁺) et/ou le Zirconium (Zr ⁴⁺) et/ou Hafnium (Hf ⁴⁺).

9. Utilisation selon la revendication 7, où le cation est l'Aluminium (Al ³⁺).

10. Utilisation selon l'une quelconque des revendications 1 à 9, où lesdites particules de silice modifiée comprennent en plus un contre-ion stabilisant anionique.

11. Utilisation selon la revendication 10 où le contre ion stabilisant est le chlore (Cl⁻).

12. Utilisation selon l'une quelconque des revendications 1 à 11, où lesdites particules colloïdales cationiques de silice sont présentes dans des quantités entre 1 et 80% en poids en matière active, plus préférentiellement entre 5% et 50% et encore plus préférentiellement entre 10% et 50% en poids par rapport au poids total de la composition.

13. Utilisation selon l'une quelconque des revendications 1 à 12, où le pH de la composition est inférieur à 7, mieux allant de 3 à 6,5.

14. Composition anti-transpirante conditionnée dans un dispositif aérosol, dans un flacon pompe, d'un roll-on, dans un dispositif muni d'une paroi ajourée ou sous forme d'un bâtonnet (stick), **caractérisée par le fait qu'**elle contient au moins une dispersion de particules colloïdales cationiques de silice telle que définie dans les revendications précédentes.

15. Composition selon la revendication 14 pressurisée et conditionnée dans un dispositif aérosol constitué par :
(A) un récipient comprenant une composition comprenant au moins une dispersion de particules colloïdales cationiques de silice telle que définie dans les revendications précédentes.
(B) au moins un agent propulseur et un moyen de distribution de la dite composition aérosol.

16. Composition anti-transpirante, **caractérisée par le fait qu'**elle contient au moins une dispersion de particules colloïdales cationiques de silice telle que définie dans les revendications précédentes et au moins un actif anti-transpirant additionnel.

17. Composition selon l'une quelconque des revendications 14 à 16, comprenant au moins un actif déodorant additionnel.

18. Composition selon l'une quelconque des revendications 14 à 17, comprenant au moins une huile émolliente siliconée ou hydrocarbonée, volatile ou non-volatile.

19. Composition selon l'une quelconque des revendications 14 à 18, contenant en plus au moins un agent de suspension.

20. Composition selon l'une quelconque des revendications 14 à 19, contenant en plus au moins une poudre organique.

21. Composition selon l'une quelconque des revendications 14 à 20, contenant au moins une phase liquide organique non-miscible dans l'eau et au moins un agent structurant de ladite phase.

22. Procédé cosmétique pour traiter la transpiration humaine, consistant à appliquer sur la surface de la peau une quantité efficace d'une dispersion de particules colloïdales cationiques de silice telle que définie dans les revendications 1 à 13 ou d'une composition telle que définie dans les revendications 14 à 21.

23. Procédé cosmétique de traitement des odeurs corporelles, consistant à appliquer sur la surface de la peau une quantité efficace d'une dispersion de particules colloïdales cationiques de silice telle que définie dans les revendications 1 à 13 ou d'une composition telle que définie dans les revendications 14 à 21.
